# EUROPEAN PATENT SPECIFICATION

(11) **EP 2 444 151 B1**
(45) Date of publication and mention of the grant of the patent: **19.06.2019**
(21) Application number: 11185295.0
(22) Date of filing: 14.10.2011
(51) Int. Cl.: B01J 23/00, B01J 23/63, B01J 37/04, B01J 21/06, A61L 2/232, A61L 2/26

(54) **Metal oxide sterilizing catalyst, and sterilizing device and system including the same**
Metalloxidsterilisierungskatalysator sowie Sterilisierungsvorrichtung und -system damit
Catalyseur de stérilisation d'oxydes métalliques, dispositif de stérilisation et système l'incluant

(30) Priority: 19.10.2010 KR 20100102095
(43) Date of publication of application: 25.04.2012
(73) Proprietor: Samsung Electronics Co., Ltd., Suwon-si, Gyeonggi-do, 443-742 (KR)
(72) Inventor: Kim, Jae Eun, Seoul (KR); Ji, Sang Min, Suwon-si Gyeonggi-do (KR); Lee, Joo Wook, Seoul (KR); Kang, Hyo Rang, Anyang-si Gyeonggi-do (KR); Yang, Ho Jung, Suwon-si Gyeonggi-do (KR); Kim, Hyun Seok, Seoul (KR); Kim, Chang Hyun, Seoul (KR)
(74) Representative: Elkington and Fife LLP

(56) References cited:
- EP-A1- 1 932 590
- EP-A1- 2 191 884
- WO-A1-2008/006748
- WO-A2-2010/028016
- FR-A3- 2 939 329
- KR-A- 20040 082 246
- US-A1- 2003 235 624

## Description

Example embodiments relate to a sterilizing device comprising a metal oxide sterilizing catalyst and a sterilizing system using the same.

Recently, various antibacterial products have been manufactured in response to microorganic contamination observed in various environments and the demands for cleaner and safer environments.

Antibacterial agents are generally characterized as either a chlorine type, an organic type, an inorganic type, etc. The number of products using inorganic type of antibacterial agents including silver has largely increased. Because silver (Ag) is known to be harmless to a human body and has excellent antibacterial effects for various microorganisms, it is used for sterilization of drinking water, kitchen utensils, various living goods, etc. Conventional antibacterial agents use the antibacterial activity of silver ions, wherein the silver ions released from the antibacterial agents directly play a role in the antibacterial activities. It is known that the released silver ions replace hydrogen atoms of a thiol group (-SH) in amino acids making up the enzyme of the microorganisms to form a sulfur-silver complex (-S-Ag). The sulfur-silver complex neutralizes the activity of the enzyme, or penetrates into a cell wall of the microorganisms to bond with DNA and disturb respiration.

US 2003/0235624 A1 discloses a bactericidal filler material formed of pyrogenic silica doped with silver or silver oxide.

WO 2010/028016 A2 discloses a method for preparing a titania dispersion by blending two different titania dispersions synthesized by a solvothermal process and a hydrolysis process. The titania dispersion may be used for decontamination purposes.

KR 2004-0082246 discloses a particulate metal oxide catalyst with antibacterial and deodorizing effect.

### SUMMARY

Example embodiments relate to a sterilizing device and a sterilizing system using the same.

Example embodiments provide a sterilizing device using a sterilizing catalyst that may be used for sterilization at room temperature, and that exhibits continual sterilizing performance.

Still other example embodiments provide a sterilizing system including a sterilizing catalyst, and a reactive oxygen species generated by an oxygen vacancy of the sterilizing catalyst, thus performing sterilization activity.

According to example embodiments, a sterilizing device comprises a coating layer including a sterilizing catalyst, the sterilizing catalyst comprising: a metal oxide lattice including a metal oxide selected from CeₓZr₁₋ₓO₂ (0<x<1), CeO-ZrO₂ and a combination thereof, and an oxygen vacancy-inducing metal that is substituted for a portion of the metal oxide lattice or substituted for a portion of the metal oxide lattice and inserted in a space between the lattice. The oxygen vacancy-inducing metal has an oxidation number lower than that of the metal of the metal oxide and is selected from silver (Ag), copper (Cu), zinc (Zn), cobalt (Co), nickel (Ni), manganese (Mn) and a combination thereof, and wherein a lattice frame of the metal oxide lattice including the metal oxide and having the substituted or substituted and inserted oxygen vacancy-inducing metal is identical to a lattice frame where the oxygen vacancy-inducing metal is not substituted or substituted and inserted.

The diameter of the oxygen vacancy-inducing metal may be smaller than the diameter of the metal in the metal oxide.

The sterilizing catalyst may produce a reactive oxygen species in a temperature range of 4°C to 30°C and in the dark.

The oxygen vacancy-inducing metal may form 0.1 to 20 wt% of the entire sterilizing catalyst.

In the sterilizing catalyst, the oxygen vacancy-inducing metal may not be ionized in an aqueous solution.

The sterilizing catalyst may exclude (i.e., not include) an additional metal selected from platinum (Pt), palladium (Pd), ruthenium (Ru), rhodium (Rh), gold (Au) and a combination thereof.

The sterilizing catalyst may be used for sterilization under a temperature of about 4°C to about 30°C and under dark conditions.

According to still other example embodiments, a sterilizing system is provided that includes the above-described sterilizing device, and a reactive oxygen species formed from the sterilizing catalyst.

The metal of the metal oxide may have a variable oxidation state, whereby the reactive oxygen species is formed from an oxygen vacancy induced by a change in the oxidation state of the metal of the metal oxide due to the introduction of the oxygen vacancy-inducing metal in the sterilizing catalyst.

Reactive oxygen species may be produced (or exist) in a temperature range of 4°C to 30°C and under dark conditions.

### BRIEF DESCRIPTION OF THE DRAWINGS

FIG. 1 is a graph showing XRD analysis results of catalyst samples obtained in Example 1, Example 2, and Comparative Example 1.
FIG. 2 is a graph showing remaining Ag amounts of catalyst samples prepared in Example 1, Example 2 and Comparative Example 2 with time, measured after adding the catalyst samples to a microorganism culture.
FIG. 3 is a graph showing electron spin resonance (ESR) measurement results of the catalyst samples prepared in Comparative Example 1, Example 1 and Example 2.
FIG. 4 is a magnified graph of the ESR measurement graph of Example 1 of FIG. 3.
FIG. 5 is a graph showing microorganism concentrations of the catalyst samples of Comparative Example 1, Comparative Example 2, Example 1 and Example 2, measured after conducting sterilization tests using each of the catalyst samples.
FIG. 6 is a graph showing the effect of dissolved oxygen on sterilization performance of the catalyst samples of Example 1 and Example 2, measured after conducting sterilization tests using each of the catalyst samples.
FIG. 7 is an illustration of a coating ball including the metal oxide sterilized catalyst according to example embodiments;
FIGS. 8 and 9 are illustrations of a filter used in a sterilizing method according to example embodiments.
FIG. 10 illustrates a sterilizing device including the sterilizing catalyst according to example embodiments.

### DETAILED DESCRIPTION

Various example embodiments will now be described more fully with reference to the accompanying drawings in which some example embodiments are shown. However, specific structural and functional details disclosed herein are merely representative for purposes of describing example embodiments. Thus, the invention may be embodied in many alternate forms and should not be construed as limited to only example embodiments set forth herein. Therefore, it should be understood that there is no intent to limit example embodiments to the particular forms disclosed, but on the contrary, example embodiments are to cover all modifications, equivalents, and alternatives falling within the scope of the invention.

Although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of example embodiments. As used herein, the term "and/or" includes any and all combinations of one or more of the associated listed items.

The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises," "comprising," "includes" and/or "including," if used herein, specify the presence of stated features, integers, steps, operations, elements and/or components, but do not preclude the presence or addition of one or more other features, integers, steps, operations, elements, components and/or groups thereof.

It should also be noted that in some alternative implementations, the functions/acts noted may occur out of the order
Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

In order to more specifically describe example embodiments, various aspects will be described in detail with reference to the attached drawings. However, the present invention is not limited to example embodiments described.
Example embodiments relate to a metal oxide sterilizing catalyst, a sterilizing device and a sterilizing system using the same.

First, a sterilizing catalyst according to example embodiments is explained. The sterilizing catalyst according to example embodiments includes a metal oxide lattice including a metal oxide, and an oxygen vacancy-inducing metal that is substituted for a portion of (i.e. integrated in) the metal oxide lattice or is substituted for a portion of the metal oxide lattice and inserted in (i.e. encompassed within) a space between the lattice.

The oxidation number of the oxygen vacancy-inducing metal is lower than the oxidation number of the metal in the metal oxide.

The frame of the metal oxide lattice is maintained by substitution or substitution and insertion of the oxygen vacancy-inducing metal without deformation. Specifically, the sterilizing catalyst has a lattice frame identical to the lattice frame of a metal oxide in which the oxygen vacancy-inducing metal is not substituted or substituted and inserted.

To maintain the lattice frame, after substitution or substitution and insertion of the oxygen vacancy-inducing metal in the metal oxide, an oxygen vacancy-inducing metal having a diameter smaller than that of the metal in the metal oxide may be selected.

Because the metal oxide is an oxide of a divalent or multivalent metal and the oxidation number of the oxygen vacancy-inducing metal is lower than the oxidation number of the metal of the metal oxide, substitution or insertion of the oxygen vacancy-inducing metal in the lattice frame of the metal oxide may induce an oxygen vacancy.

The induced oxygen vacancy may cause adsorption of water, oxygen, etc. due to its electron-attracting property, and the adsorbed water or oxygen may be oxidized to a reactive oxygen species (e.g., a superoxide anion radical (O₂⁻), a hydroxide radical (OH), hydrogen peroxide H₂O₂, etc.). The produced reactive oxygen species may oxidize microorganisms to perform sterilization. Alternatively, the oxygen vacancy of the metal oxide may directly take up electrons from microorganisms thus performing sterilization by oxidation.

As explained, because sterilization is performed by a reactive oxygen species rather than by an oxygen vacancy-inducing metal or other reactive metal(s) released from the sterilizing catalyst, sterilization performance of the sterilizing catalyst may be stably maintained for a longer period of time, and a semi-permanent antibacterial life-span may be realized.

Thus, the oxygen vacancy-inducing metal may not be substantially released from the sterilizing catalyst during sterilization (i.e., the oxygen vacancy-inducing metal may be retained).

In addition, the oxygen vacancy-inducing metal may not be ionized and released in an aqueous solution, and is firmly maintained integrated or interstitially in the lattice of the metal oxide.

Because the sterilizing catalyst according to example embodiments may perform sterilization using a reactive oxygen species generated from an oxygen vacancy that is induced by an oxygen vacancy-inducing metal substituted or inserted in the lattice of the metal oxide, it may not be required to further include an active metal (e.g., platinum (Pt), palladium (Pd), ruthenium (Ru), rhodium (Rh), gold (Au) or a similar metal) in the metal oxide, but is not limited thereto. Thus, the sterilizing catalyst may, or may not, include the active metal.

Because specific conditions including additional energy supply (e.g., high temperature or light) may not be required to generate reactive oxygen species from the induced oxygen vacancy, sterilization may be performed under (or in) a dark condition, or at low temperature to room temperature. Specifically, because the generation of reactive oxygen species is not related to additional energy supply (e.g., temperature condition), the reactive oxygen species may exhibit an excellent sterilizing effect at any temperature, and similarly, the reactive oxygen species may be generated even under (or in) a substantially dark condition wherein solar light is not provided to exhibit an excellent sterilizing effect. As explained, because an excellent sterilizing effect may be obtained at low temperature to room temperature as well as at a high temperature, the reactive oxygen species may be produced for example at a temperature of about 4°C to about 30°C and under dark conditions.

Specific examples of the oxygen vacancy-inducing metal may include Ag, Cu, Zn, Co, Mn and a combination thereof.

According to example embodiments, the sterilizing catalyst may include the oxygen vacancy-inducing metal in a content of about 0.1 to 20 wt% of the entire sterilizing catalyst. If the oxygen vacancy inducing metal is included within the above range, the structure of the metal oxide may be firmly maintained and the oxygen vacancy degree may be increased.

The sterilizing catalyst may be applied for a product or system requiring antibacterial, sterilizing performance.

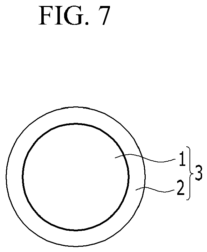

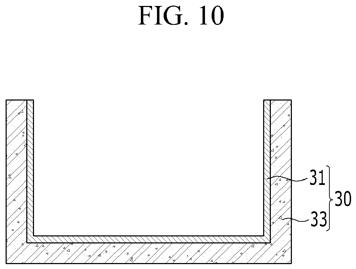

Specific examples of the sterilizing device may include systems requiring sterilized water (e.g., water purifiers, water reservoirs, humidifiers, etc.), and systems requiring sterilization of air (e.g., air purifiers, various household items requiring a sterilizing function, etc.). As an example, FIG. 10 illustrates a water reservoir 30 as one example of the sterilizing device according to example embodiments. The water reservoir 30 may include a coating layer 31 and a container 33. The coating layer 31 may include the sterilizing catalyst according to example embodiments. The coating layer may be prepared by a known coating layer manufacturing method without specific limitation, and for example, it may be manufactured by preparing an aqueous solution composition including the sterilizing catalyst, coating the aqueous solution composition on a subject, and drying. For example, as illustrated in FIG. 7, a coating ball 3 may be formed by coating the coating layer 2 on an alumina ball, and prepared in the form of a ceramic ball 1.

According to still other example embodiments, a sterilizing system is provided that includes the above-described sterilizing catalyst and a reactive oxygen species induced from the sterilizing catalyst. Specifically, the sterilizing catalyst making up the sterilizing system includes an oxygen vacancy-inducing metal that is substituted for (i.e. integrated in) a metal lattice of a metal oxide, or substituted for and inserted in (i.e. encompassed within) a space between the lattice. The metal oxide is an oxide of the divalent or multivalent metals specified above, and the oxidation number of the oxygen vacancy-inducing metal is lower than the oxidation number of the metal of the metal oxide.

The reactive oxygen species may be produced by an oxygen vacancy that is caused by a change in the oxidation state of a metal making up a lattice of the sterilizing catalyst due to introduction of an oxygen vacancy-inducing metal having a smaller oxidation number than a metal oxide in the sterilizing catalyst.

As explained above, generation of the reactive oxygen species may not require additional energy supply, so sterilization may be performed at low temperature to room temperature, or under dark conditions.

Specifically, because generation of the reactive oxygen species is not related to additional energy supply (e.g., a temperature condition), the sterilizing catalyst may generate a reactive oxygen species at any temperature that exhibits an excellent sterilizing effect, and similarly, it may generate a reactive oxygen species even under a dark condition that exhibits an excellent sterilizing effect. As explained, because an excellent sterilizing effect may be obtained at a low temperature to room temperature as well as at a high temperature, the reactive oxygen species may be produced for example at temperature of about 4°C to about 30°C and under dark condition.

Hereinafter, a method of manufacturing a sterilizing catalyst is explained in detail.

The sterilizing catalyst may be prepared by various methods (e.g., evaporation-induced self-assembly, co-precipitation, etc.).

According to the evaporation-induced self assembly, a metal oxide precursor and an oxygen vacancy inducing metal precursor are added to a solvent to prepare a mixed solution, the mixed solution is then dried and aged, and the resultant product is baked to form an oxide catalyst with a porous structure.

As the solvent mixed with the precursors, an alcohol-type solvent (e.g., methanol, ethanol, etc.) may be used, and an acid (e.g., a hydrochloric acid, a nitric acid, an acetic acid, etc.) may be mixed therewith. The content of the solvent may not be specifically limited, but it may be included in the content of about 0.1 to about 40 parts by weight based on 100 parts by weight of the oxide precursor.

The metal oxide precursor and the oxygen vacancy-inducing metal precursor are mixed with the solvent to form a mixed solution, and a structure-forming agent may be further added thereto. The structure-forming agent may provide a backbone of the metal oxide, and, for example, a neutral surfactant may be used. Specific examples of the neutral surfactant may include a polyethylene oxide/polypropylene oxide/polyethylene oxide (PEO/PPO/PEO) triblock copolymer of the product name Pluronic F108, F127, etc.

As the oxygen vacancy-inducing metal precursor used in the manufacturing process, a compound (e.g., an alkoxide, a halide, a nitrate, a chlorate, a sulfate, or an acetate) which includes a metal selected from Ag, Cu, Zn, Co, and Mn may be used.

As the metal oxide precursor used in the manufacturing process, a compound (e.g., an alkoxide, a halide, a nitrate, a chlorate, a sulfate, or an acetate) which includes at least one metal selected from Ce and Zr may be used.

In the manufacturing process, two or more types of metal oxide precursors are used, and a composite metal oxide is formed. A support for the composite metal oxide includes CeₓZr₁₋ₓO₂ (0<x<1), CeO-ZrO₂ or a combination thereof.

The mixed solution including a solvent, a catalyst metal precursor, and a metal oxide precursor, and if necessary, an acid or a structure-directing agent may be agitated at room temperature (about 24°C) for about 0.1 to about 10 hours to homogenize each component.

The obtained mixed solution may be allowed to stand at room temperature (about 24°C) and atmospheric pressure (about 1 atm) for about 1 to about 100 hours to remove a volatile solvent component in the mixed solution. The standing time may not be specifically limited as long as it may remove the volatile solvent component.

The product obtained after removing the solvent component, if necessary, may be subjected to an aging process. The aging process increases the bonding degree between atoms forming the structure, and it may be conducted at an elevated temperature of about 30 to about 100°C for about 6 to about 48 hours.

Next, the product passing the aging process may be subjected to a baking process whereby each precursor may be transformed to an oxide. The baking process may be conducted in the atmosphere, in the temperature range of from about 300°C to about 1000°C, specifically from about 350°C to about 600°C, for about 0.1 to about 30 hours, specifically for about 1 to about 10 hours.

Each precursor may be transformed to a metal oxide by the baking process, during which the metal oxide forms a mesopore structure, and the oxygen vacancy inducing metal becomes substituted and/or inserted in the backbone of the metal oxide.

According to co-precipitation, which is another method for manufacturing the sterilizing catalyst, a basic aqueous solution is added to a water dispersion including the precursors to form a precipitate in the form of a hydroxide, and then the precipitate is filtered and washed and the resultant product is baked to prepare a sterilizing catalyst. The type of precursor and baking conditions are identical to the evaporation-induced self assembly.

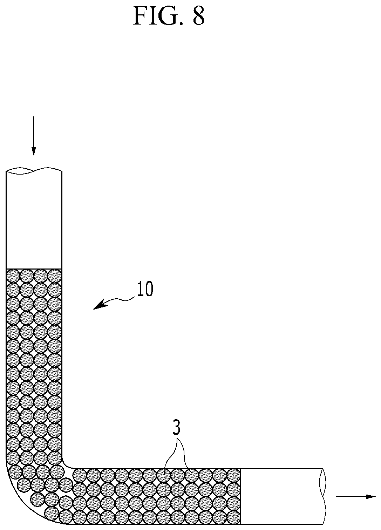

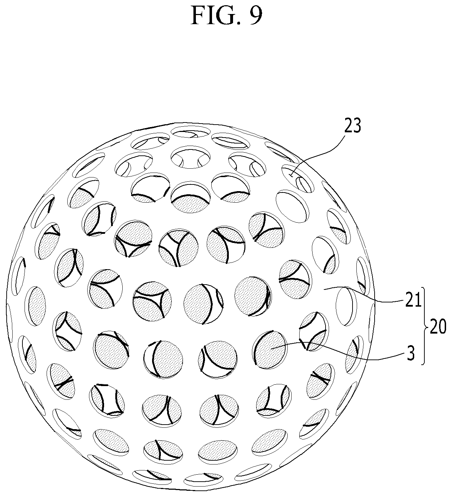

FIGS. 8 and 9 are illustrations of a filter used in the above-described sterilizing method according to example embodiments. In FIG. 8, a filter 10 is filled with the coating ball 3. Water may be purified in the filter 10 in the direction of arrows. It is noted that the shape of the filter 10 is not limited to the shape depicted in FIG. 8, but rather is provided for illustration purposes. In FIG. 9, a filter 20 may be in the shape of a relatively large hollow ball 21 including the coating balls 3. The hollow ball 21 may have a plurality of holes 23 on its surface. The plurality of holes may prevent or reduce the movement of the coating balls 3 therethrough, because the diameter of the plurality of holes 23 is smaller than the diameter of the coating ball 3.

Among the manufacturing processes, the evaporation-induced self assembly is favorable for obtaining a uniform catalyst having a pore size of narrow distribution, and the co-precipitation is favorable for obtaining a catalyst having various pore sizes.

As explained, because manufacturing of the sterilizing catalyst may be conducted simply by baking the precursors, it may be prepared with a low cost, thus enabling manufacture of a high efficiency and low cost composite catalyst. Hereinafter, the example embodiments are illustrated in more detail with reference to the following examples. However, the following are exemplary embodiments and are not limiting.

### EXAMPLES

### Example 1: Preparation of Ce_{0.85}Ag_{0.05}Zr_{0.1}O_{1.925}

An oxygen vacancy inducing metal (Ag) is substituted (or integrated) in a metal oxide lattice to synthesize a Ce_{0.85}Ag_{0.05}Zr_{0.1}O_{1.925} sterilizing catalyst as follows.
ethanol: 30ml
hydrochloric acid: 1.97ml
Pluronic: 4.6g
(PEO/PPO/PEO triblock copolymer)
acetic acid: 2.4g
Ce(NO₃)₃: 9.23g
AgNO₃: 0.21g
Zr(OC₄H₉)₄: 1.2g

The above components are introduced into a beaker and agitated at room temperature for 5 hours. Subsequently, the mixture is dried at room temperature for 2 days and aged at 338K for 12 hours. The resultant product is baked at 673K for 5 hours to prepare a Ce_{0.85}Ag_{0.05}Zr_{0.1}O_{1.95} sterilizing catalyst. The content of the metal precursor used is such that the mole ratio of Ce NO₃₃:AgNO₃:Zr (OC₄H₉)₄ is about 0.85:0.05:0.1, thus stoichiometrically substituting Ag in the oxide backbone.

### Example 2: Preparation of Ag_{0.05}Ce_{0.9}Zr_{0.1}O₂

An antibacterial metal is substituted and inserted (i.e., integrated and encompassed) in a metal oxide lattice to synthesize a Ag_{0.05_}Ce_{0.9}Zr_{0.1}O₂ sterilizing catalyst as follows.
ethanol: 30ml
hydrochloric acid: 1.97ml
Pluronic F127: 4.6g
acetic acid: 2.4g
Ce(NO₃)₃: 9.77g
AgNO₃: 0.21g
Zr(OBu)₄: 1.2g

The above components are introduced into a beaker and agitated at room temperature for 5 hours. Subsequently, the mixture is dried at room temperature for 2 days and aged at 338K for 12 hours. The resultant product is baked at 673K for 5 hours to prepare a Ag_{0.05_}Ce_{0.9}Zr_{0.1}O₂ sterilizing catalyst.

The content of the metal precursor used is such that the mole ratio of Ce NO₃₃:AgNO₃:Zr (OC₄H₉)₄ is about 0.9:0.05:0.1, thus inserting excessive Ag in the space between metal oxide lattices. Because the prepared sterilizing catalyst includes substituted and inserted Ag ions, it is indicated as Ag_{0.05_}Ce_{0.9}Zr_{0.1}O₂ so as to distinguish it from the sterilizing catalyst of Example 1 including only substituted Ag.

### Comparative Example 1: Preparation of Ce_{0.9}Zr_{0.1} O₂

ethanol: 30ml
hydrochloric acid: 1.97ml (or nitric acid having the same mole ratio)
Pluronic F127: 4.6g
acetic acid: 2.4g
Ce(NO₃)₃: 9.77g (metal precursor mole ratio: 0.9)
Zr(OBu)₄: 1.2g (metal precursor mole ratio: 0.1)

The above components are introduced into a beaker, and agitated at room temperature for 5 hours. Subsequently, the mixture is dried at room temperature for 2 days and aged at 338K for 12 hours. The resultant product is baked at 673K for 5 hours to prepare a Ce_{0.9}Zr_{0.1}O₂ metal oxide catalyst.

### Comparative Example 2: Preparation of Aq-zeolite

Na-Y zeolite (Tosoh Corporation) 2 g
0.1M AgNO₃ 50ml

2g of a Na-Y zeolite is baked for 4 hours while maintaining the temperature at 400°C. Then, the baked Na-Y zeolite is introduced into 50 ml of an AgNO₃ solution of a 0.1M concentration, and agitated for 5 hours while maintaining the temperature at 60°C to conduct ion exchange. The ion-exchanged Ag-Y zeolite is filtered and washed with deionized water. The washed Ag-Y zeolite is dried for 16 hours while maintaining the temperature at 110°C. The dried Ag-Y zeolite is introduced into a heating furnace and baked for 4 hours while maintaining the temperature at 400°C.

### Experimental Example 1

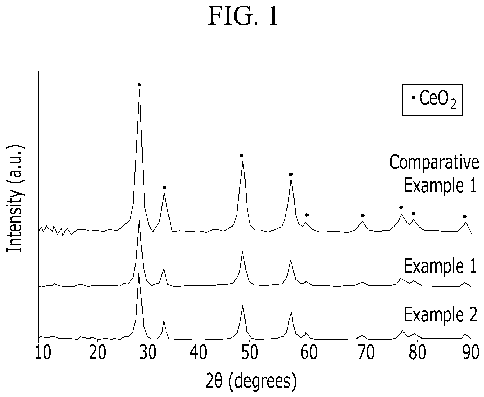

FIG. 1 shows XRD analysis results of the catalyst samples obtained in Example 1, Example 2 and Comparative Example 1. From FIG. 1, it is observed that all the sterilizing catalysts of Example 1, Example 2, and Comparative Example 1 show the same peaks as a CeO₂ lattice without peaks of other atoms. Thus, it can be seen that an antibacterial metal element (Ag) is stably substituted or inserted in the CeO₂ lattice in the sterilizing catalysts of Example 1 and Example 2.

### Experimental Example 2: Silver ion release

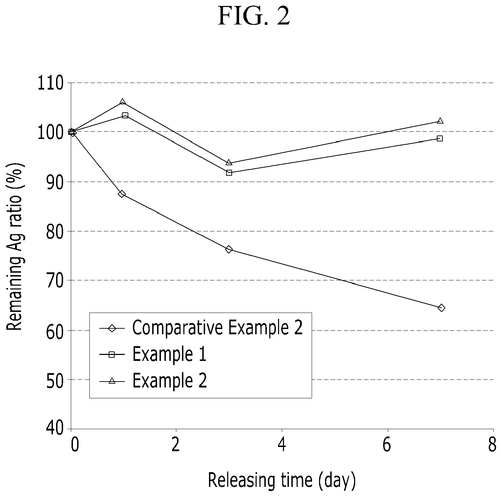

To determine whether or not silver ions are released when the sterilizing catalysts prepared in Example 1, Example 2 and Comparative Example 2 are exposed to an aqueous solution, a release test is performed. 20 ml of an LB (Luria Bertani) solution (microorganism culture) of a 0.25wt% concentration is introduced into a 50 mL Falcon tube, and then, 100 mg of each of the sterilizing catalysts of Example 1, Example 2 and Comparative Example 2 are introduced therein and disposed in a shaking incubator. The elution test is conducted at 25°C, 150 rpm for 1 week, and the sample is taken out respectively at 1, 3, and 7 days. The sample is centrifuged at 7000 rpm for 10 minutes and then vacuum-dried at 90°C. The Ag content change of each sample before and after the release test is observed by ICP-AES (inductively coupled plasma - atomic emission spectrometer) analysis, and the results are shown in FIG. 2.

As a result of release test, it is observed that the Ag content remarkably decreases with time lapse in the sterilizing catalyst of Comparative Example 2 (about 65% of initial value after 7 days), while a significant change is not observed in the sterilizing catalysts of Example 1 and Example 2. Therefore, it is confirmed that Ag+ is not substantially released in the sterilizing catalysts of Example 1 and Example 2.

### Experimental Example 2-1 :Silver ion release

20 ml of an LB (Luria Bertani) solution (microorganism culture) of a 0.25wt% concentration is introduced into a 50 mL Falcon tube, and then each catalyst sample of Example 1 and Example 2 is introduced therein in the concentrations as described in the following Table 1 and disposed in a shaking incubator. The mixture is agitated at 25°C, 150 rpm for 24 hours, and silver ion concentration of the microorganism culture to which the sample is added is measured and described in the following Table 1. Silver ions are analyzed by anodic stripping voltammetry, wherein a silver ion concentration of 0 ppb means less than 10 ppb because the detection limit is 10 ppb.

**TABLE 1**

| | **CONCENTRATION OF SAMPLE INTRODUCED IN THE CULTURE (PPB)** | **[AG+] (PPB)** |
|---|---|---|
| **EXAMPLE 1** | 100,000 | 0 |
| | 10,000 | 0 |
| | 5000 | 0 |
| **EXAMPLE 2** | 100,000 | 0 |
| | 10,000 | 0 |
| | 5000 | 0 |

From the results of Table 1, it is confirmed that the release amounts of silver ions are insignificant in the sterilizing catalysts of Example 1 and Example 2.

### Experimental Example 3: Reactive oxygen species

For the catalyst samples prepared in Comparative Example 1, Example 1 and Example 2, radicals are detected by electron spin resonance (ESR, JES-TE200, JEOL, Japan) analysis to confirm generation of reactive oxygen species.

As a spin-trapping agent, DMPO (5,5-dimethyl-1-pyrroline N-oxide, sigma) is used, and ESR analysis conditions are as follows: temperature of 25°C, frequency of 9.4 GHz, scan range of 100G, field set of 3410 G (341 mT), time constant of 0.3 s, scan time of 4 min, modulation amplitude of 4, modulation frequency of 100 kHz, and microwave power of 1 mW. A test solution is prepared by introducing 10 mg of the sterilizing catalyst and 25 ul of a DMPO solution (final concentration 0.5 mM) into 500 ul of distilled water, and the solution is introduced into an ESR tube and analyzed.

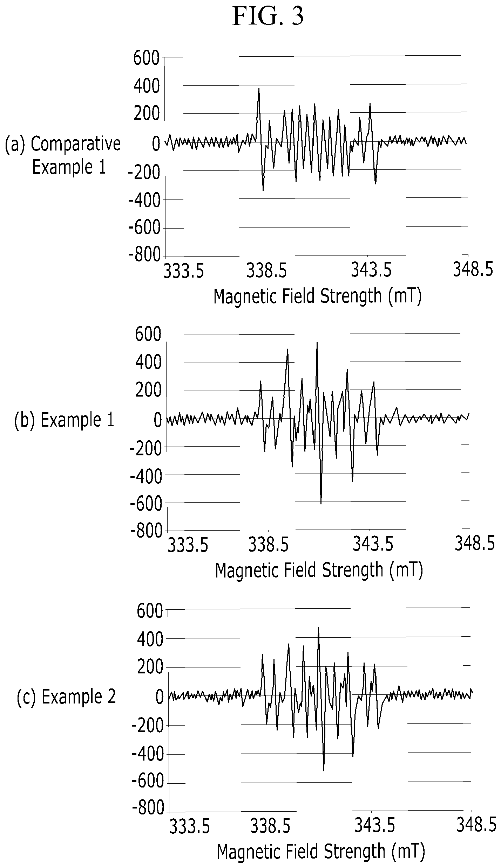

The ESR measurement results are described in FIG. 3. It is shown that radicals are not substantially detected in Comparative Example 1 of FIG. 3(a), and radicals are detected in Example 1 of FIG. 3(b) and Example 2 of FIG. 3(c). From these results, it can be seen that reactive oxygen species are not produced in Comparative Example 1, while reactive oxygen species are produced in Example 1 and Example 2.

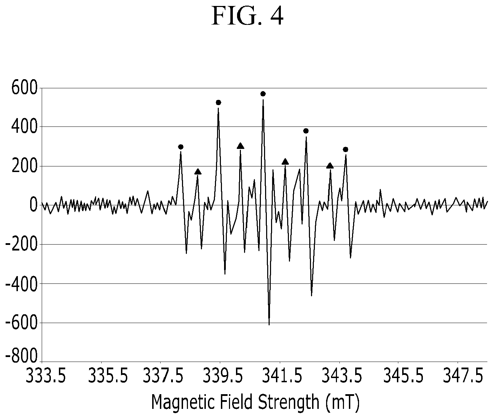

FIG. 4 is a magnification of the graph of Example 1 of FIG. 3(b), wherein the ratio of areas of 4 peaks indicated as triangles is 1:2:2:1, and the peak interval is regular at 15 G (1.5 mT), thus the peaks are analyzed as typical peaks of hydroxide radicals. Therefore, it can be seen that multiple reactive oxygen species including hydroxide radicals are generated.

### Experimental Example 4

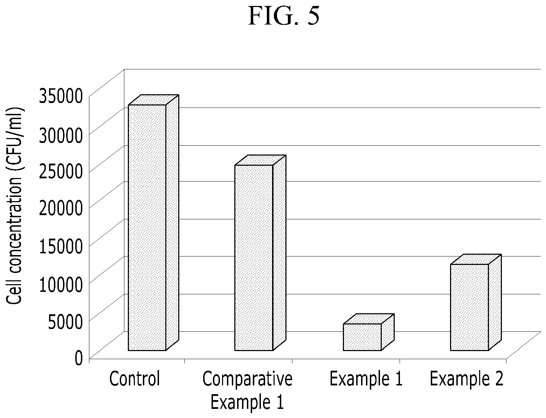

For a control of E. coli having an initial concentration of 32,000 CFU/ml and the sterilizing catalysts of Comparative Example 1, Example 1, and Example 2, sterilizing tests are conducted at 25°C with a catalyst concentration of 1mg/20ml, and the results are shown in FIG. 5.

It is observed that microorganisms are grown to increase the concentration in the control where no sterilizing catalyst is introduced, while Comparative Example 1 exhibits weak sterilizing performance (about a 20% decrease in microorganism concentration). On the contrary, it is confirmed that about 90% and about 70% of the microorganisms are respectively sterilized in Example 1 and Example 2, indicating that sterilizing performance is improved.

### Experimental Example 5

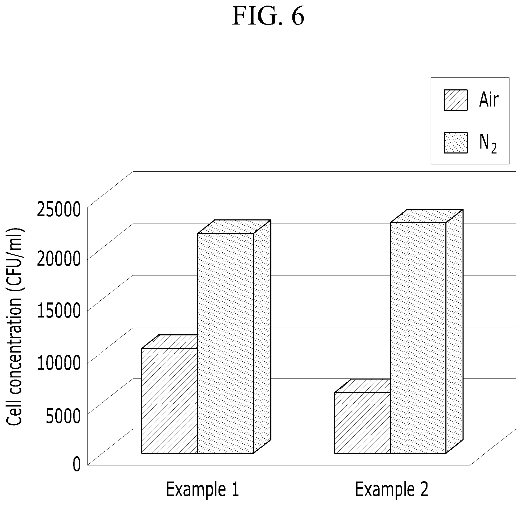

For E. coli of an initial concentration of 32,000 CFU/ml and the sterilizing catalysts of Example 1 and Example 2, sterilizing tests are conducted at 25°C with the catalyst concentration of 1mg/20ml, respectively, under an air condition where air is added by agitation and under a N₂ condition wherein dissolved oxygen is decreased by bubbling. The results are shown in FIG. 6.

From FIG. 6, it is observed that microorganism sterilizing performance is significantly different under an N₂ condition compared to an air condition. Therefore, it can be seen that reactive oxygen species are very important in the sterilizing mechanism of the sterilizing catalysts of Example 1 and Example 2.

### Experimental Example 6

To confirm the sterilizing effect according to silver ion concentration, test solutions are prepared with a AgNO₃ reagent so as to have silver ion concentrations described in the left column of the following Table 2. 20 ml of an LB solution (microorganism culture medium) of a 0.25 wt% concentration is introduced into a 50mL Falcon tube, and then the AgNO₃ reagent is added with each silver ion concentration of Table 2, and sterilizing is conducted at 25°C. An initial concentration of E.coli is 10,000 CFU/ml. Further, each E. coli concentration is measured and described in the following Table 2.

**TABLE 2**

| **[Ag+] (ppb)** | **E. coli (CFU/mL)** |
|---|---|
| 0 | 310,000,000 |
| 1 | 370,000,000 |
| 5 | 54,500,000 |
| 10 | 14,500 |
| 15 | 140 |
| 20 | 20 |

From the results of Table 2, it can be seen that at least about 15 ppb or more of silver ion release concentration is required for use as a sterilizing catalyst. Comparing these results with the sterilizing effects of Example 1 and Example 2 as shown in Experimental Example 2, Experimental Example 4, and Experimental Example 5, it can be seen that the sterilizing effects of Example 1 and Example 2 are not derived from the silver ion release concentration because Example 1 and Example 2 exhibit sterilizing effects in spite of silver ion release concentrations of less than 10 ppb. Specifically, the sterilizing effects of Example 1 and Example 2 are derived from reactive oxygen species, and thus excellent sterilizing effects are obtained in spite of a low silver ion elution concentration.

## Claims

1. A sterilizing device comprising a coating layer including a sterilizing catalyst, the sterilizing catalyst comprising:
a metal oxide lattice including a metal oxide selected from CeₓZr₁₋ₓO₂ (0<x<1), CeO-ZrO₂ and a combination thereof, and
an oxygen vacancy-inducing metal that is substituted for a portion of the metal oxide lattice or substituted for a portion of the metal oxide lattice and inserted in a space between the lattice,
wherein the oxygen vacancy-inducing metal has an oxidation number lower than that of the metal of the metal oxide and is selected from silver (Ag), copper (Cu), zinc (Zn), cobalt (Co), nickel (Ni), manganese (Mn) and a combination thereof, and
wherein a lattice frame of the metal oxide lattice including the metal oxide and having the substituted or substituted and inserted oxygen vacancy-inducing metal is identical to a lattice frame where the oxygen vacancy-inducing metal is not substituted or substituted and inserted.

2. The sterilizing device of claim 1, wherein the oxygen vacancy-inducing metal comprises silver (Ag).

3. The sterilizing device of claims 1 or 2, wherein the sterilizing catalyst is capable of producing a reactive oxygen species under a temperature of 4°C to 30°C and under dark conditions.

4. The sterilizing device of any of claims 1-3, wherein the oxygen vacancy-inducing metal forms 0.1 to 20 wt% of the entire sterilizing catalyst.

5. The sterilizing device of any of claims 1-4, wherein the oxygen vacancy-inducing metal is not ionized in an aqueous solution.

6. The sterilizing device of any of claims 1-5, wherein an additional metal selected from platinum (Pt), palladium (Pd), ruthenium (Ru), rhodium (Rh), gold (Au) and a combination thereof is not present in the sterilizing catalyst.

7. The sterilizing device of claim 1, wherein the sterilizing catalyst is capable of sterilizing in a temperature range of 4 to 30°C and under dark conditions.

8. A sterilizing system, comprising:
the sterilizing device according to any of claims 1-7; and
a reactive oxygen species formed from the sterilizing catalyst.

9. The sterilizing system of claim 8, wherein the divalent or multivalent metal has a variable oxidation state, whereby the reactive oxygen species is formed from an oxygen vacancy induced by a change in the oxidation state of the divalent or multivalent metal due to the introduction of the oxygen vacancy-inducing metal in the sterilizing catalyst.

10. The sterilizing system of claim 8 or 9, wherein the reactive oxygen species exists in a temperature range of 4°C to 30°C and under dark conditions.

## Patentansprüche

1. Sterilisierungsvorrichtung, umfassend eine Beschichtungsschicht, die einen Sterilisierungskatalysator beinhaltet, wobei der Sterilisierungskatalysator Folgendes umfasst:
ein Metalloxidgitter, das ein Metalloxid beinhaltet, das aus CeₓZr₁₋ₓO₂(0<x<1), CeO-ZrO₂ und einer Kombination davon ausgewählt ist, und
ein sauerstoffleerstelleninduzierendes Metall, das für einen Abschnitt des Metalloxidgitters substituiert ist oder für einen Abschnitt des Metalloxidgitters substituiert und in einen Raum zwischen dem Gitter eingesetzt ist,
wobei das sauerstoffleerstelleninduzierende Metall eine Oxidationszahl aufweist, die unter der von dem Metall des Metalloxids liegt, und aus Silber (Ag), Kupfer (Cu), Zink (Zn), Kobalt (Co), Nickel (Ni), Mangan (Mn) und einer Kombination davon ausgewählt ist und
wobei ein Gitterrahmen des Metalloxidgitters, welches das Metalloxid beinhaltet und das substituierte oder substituierte und eingesetzte sauerstoffleerstelleninduzierende Metall aufweist, identisch mit einem Gitterrahmen ist, bei dem das sauerstoffleerstelleninduzierende Metall nicht substituiert oder substituiert und eingesetzt ist.

2. Sterilisierungsvorrichtung nach Anspruch 1, wobei das sauerstoffleerstelleninduzierende Metall Silber (Ag) umfasst.

3. Sterilisierungsvorrichtung nach Anspruch 1 oder 2, wobei der Sterilisierungskatalysator in der Lage ist, bei einer Temperatur von 4°C bis 30°C und bei dunklen Bedingungen eine reaktive Sauerstoffspezies zu erzeugen.

4. Sterilisierungsvorrichtung nach einem der Ansprüche 1-3, wobei das sauerstoffleerstelleninduzierende Metall 0,1 bis 20 Gew.-% des gesamten Sterilisierungskatalysators bildet.

5. Sterilisierungsvorrichtung nach einem der Ansprüche 1-4, wobei das sauerstoffleerstelleninduzierende Metall in einer wässrigen Lösung nicht ionisiert ist.

6. Sterilisierungsvorrichtung nach einem der Ansprüche 1-5, wobei ein zusätzliches Metall, das aus Platin (Pt), Palladium (Pd), Ruthenium (Ru), Rhodium (Rh), Gold (Au) und einer Kombination davon ausgewählt ist, nicht in dem Sterilisierungskatalysator vorhanden ist.

7. Sterilisierungsvorrichtung nach Anspruch 1, wobei der Sterilisierungskatalysator in der Lage ist, in einem Temperaturbereich von 4 bis 30°C und bei dunklen Bedingungen zu sterilisieren.

8. Sterilisierungssystem, umfassend:
die Sterilisierungsvorrichtung nach einem der Ansprüche 1-7; und
eine reaktive Sauerstoffspezies, die aus dem Sterilisierungskatalysator gebildet wurde.

9. Sterilisierungssystem nach Anspruch 8, wobei das zweiwertige oder mehrwertige Metall einen variablen Sauerstoffzustand aufweist, wobei die reaktive Sauerstoffspezies aus einer Sauerstoffleerstelle gebildet ist, die aufgrund der Einführung des sauerstoffleerstelleninduzierenden Metalls in den Sterilisierungskatalysator durch eine Änderung des Sauerstoffzustandes des zweiwertigen oder mehrwertigen Metalls induziert wurde.

10. Sterilisierungssystem nach Anspruch 8 oder 9, wobei die reaktive Sauerstoffspezies in einem Temperaturbereich von 4°C bis 30°C und bei dunklen Bedingungen vorhanden ist.

## Revendications

1. Dispositif de stérilisation comprenant une couche de revêtement incluant un catalyseur de stérilisation, le catalyseur de stérilisation comprenant :
un réseau d'oxyde de métal incluant un oxyde de métal sélectionné parmi CeₓZr₁₋ₓO₂ (0 < x < 1), CeO-ZrO₂ et une combinaison de ceux-ci, et
un métal induisant une lacune d'oxygène qui est substitué à une partie du réseau d'oxyde de métal ou substitué à une partie du réseau d'oxyde de métal et inséré dans un espace entre le réseau,
dans lequel le métal induisant une lacune d'oxygène a un degré d'oxydation inférieur à celui du métal de l'oxyde de métal et est sélectionné parmi l'argent (Ag), le cuivre (CU), le zinc (Zn), le cobalt (Co), le nickel (Ni), le manganèse (Mn) et une combinaison de ceux-ci, et
dans lequel un cadre de réseau du réseau d'oxyde de métal incluant l'oxyde de métal et ayant le métal induisant une lacune d'oxygène substitué ou substitué et inséré est identique à un cadre de réseau où le métal induisant une lacune d'oxygène n'est pas substitué ou substitué et inséré.

2. Dispositif de stérilisation selon la revendication 1, dans lequel le métal induisant une lacune d'oxygène comprend de l'argent (Ag).

3. Dispositif de stérilisation selon les revendications 1 ou 2, dans lequel le catalyseur de stérilisation est capable de produire une espèce réactive de l'oxygène à une température de 4 °C à 30 °C et dans des conditions d'obscurité.

4. Dispositif de stérilisation selon l'une quelconque des revendications 1 à 3, dans lequel le métal induisant une lacune d'oxygène forme 0,1 à 20 % en poids du catalyseur de stérilisation total.

5. Dispositif de stérilisation selon l'une quelconque des revendications 1 à 4, dans lequel le métal induisant une lacune d'oxygène n'est pas ionisé dans une solution aqueuse.

6. Dispositif de stérilisation selon l'une quelconque des revendications 1 à 5, dans lequel un métal supplémentaire sélectionné parmi le platine (Pt), le palladium (Pd), le ruthénium (Ru), le rhodium (Rh), l'or (Au) et une combinaison de ceux-ci n'est pas présent dans le catalyseur de stérilisation.

7. Dispositif de stérilisation selon la revendication 1, dans lequel le catalyseur de stérilisation est capable de stérilisation dans une plage de température de 4 °C à 30 °C et dans des conditions d'obscurité.

8. Système de stérilisation, comprenant :
le dispositif de stérilisation selon l'une quelconque des revendications 1 à 7 ; et
une espèce réactive de l'oxygène formée à partir du catalyseur de stérilisation.

9. Système de stérilisation selon la revendication 8, dans lequel le métal divalent ou multivalent a un état d'oxydation variable, moyennant quoi l'espèce réactive de l'oxygène est formée à partir d'une lacune d'oxygène induite par un changement de l'état d'oxydation du métal divalent ou multivalent dû à l'introduction du métal induisant une lacune d'oxygène dans le catalyseur de stérilisation.

10. Système de stérilisation selon la revendication 8 ou 9, dans lequel l'espèce réactive de l'oxygène existe dans une plage de température de 4 °C à 30 °C et dans des conditions d'obscurité.
